# EUROPÄISCHE PATENTANMELDUNG

(11) **EP 4 609 959 A1**
(43) Veröffentlichungstag der Anmeldung: **03.09.2025**
(21) Anmeldenummer: 25160284.3
(22) Anmeldetag: 26.02.2025
(51) Int. Cl.: B05B 17/06, B05B 7/00

(54) **AKUSTOFLUIDISCHE VORRICHTUNG ZUR ERZEUGUNG VON AEROSOLEN**

(30) Priorität: 01.03.2024 DE 102024105991
(71) Anmelder: Leibniz-Institut für Festkörper- und Werkstoffforschung Dresden e.V., 01069 Dresden (DE)
(72) Erfinder: Frozanpoor, Iman, 01187 Dresden (DE); Roudini, Mehrzad, 04109 Leipzig (DE); Alsaadawi, Yara, 01069 Dresden (DE); Winkler, Andreas, 01187 Dresden (DE)
(74) Vertreter: Rauschenbach, Marion

(57) **Zusammenfassung**

Die Erfindung betrifft den Bereich der Mikrosystemtechnik und betrifft eine akustofluidische Vorrichtung, die beispielsweise in Vorrichtungen zum Drucken eingesetzt werden kann.

Die Aufgabe besteht in der Angabe einer akustofluidische Vorrichtung, die geringe Abmessungen aufweist und in Geräte zur Abscheidung von Aerosolen integriert werden kann und durch einen einfachen Aufbau einen gerichteten Aerosolstrom mit einstellbaren Aerosoleigenschaften realisiert.

Die Aufgabe wird gelöst durch eine akustofluidische Vorrichtung, die mindestens enthält, mindestens eine Aerosolauslasskomponente (9) und mindestens eine Halterkomponente (3) und mindestens je eine Zu- und/oder Ableitung für Gase (12, 17), Flüssigkeiten (15) und Wärme (14), mindestens eine elektrische Leitung und/oder elektrische Signalleitung (16) und mindestens eine elektrische Hochfrequenzsignalleitung (6) und mindestens eine Komponente (2) zum Wärmetransport und mindestens einen Temperatursensor (4) und mindestens eine mikroakustische Komponente (5) und mindestens eine Komponente (8) zur Übertragung eines Hochfrequenzsignals und mindestens eine Komponente (7) für den Flüssigkeitstransport und mindestens eine Transportgaskomponente (10) und mindestens eine Komponente (13) zur Positionierung und/oder mechanischen Fixierung.

## Beschreibung

Die Erfindung betrifft die Bereiche Mikrosystemtechnik, Mikrofluidik und Mikroakustik und betrifft eine akustofluidische Vorrichtung zur Erzeugung von Aerosolen, die z.B. in Vorrichtungen oder Systemen zur Abscheidung von Aerosolen oder in Vorrichtungen und Druckköpfen zum Drucken von bzw. mittels Aerosolen (Aerosolstrahldruck) oder in chemischen oder physikalischen Systemen zur Materialabscheidung, zur Materialsynthese oder zum Materialätzen eingesetzt werden kann, wie Atomlagenabscheidungssystemen (ALD), Atomlagenätzsystemen (ALE), physikalischen Dampfabscheidungssystemen (PVD), chemischen Synthesesystemen oder chemischen Dampfabscheidungssystemen (CVD), Pyrolysesystemen, sowie Aerosolquellen für Befeuchter, Sprühtrocknung und Sprühkühlung verwendet werden können und z.B. in den Bereichen Biowissenschaften, Bioanalytik, Biochemie, Chemie, Medizintechnik, Materialwissenschaft, Dünnschichttechnologie, Beschichtungstechnik, oder in der Druckindustrie eingesetzt und angewendet werden können.

Die Mikrosystemtechnik ist ein Zweig der Mikrotechnik und befasst sich mit der Lehre, Forschung, Entwicklung und Produktion von Mikrosystemen. So werden beispielsweise mikromechanische oder mikrooptische Komponenten mit mikroelektronischen Schaltkreisen in einem komplexen System kombiniert und integriert. Sensoren, Aktoren und Datenverarbeitung arbeiten in Mikrosystemen zusammen. Prozesse und Methoden aus verschiedenen Mikrotechnologien werden daher kombiniert, um komplexe Mikrosysteme herzustellen. Dazu gehören Mikromechanik, Mikrofluidik, Bioelektronik, Mikrooptik und die Mikroelektronik (Datenverarbeitung, elektronische Schnittstellen) selbst. Die verwendeten Herstellungsverfahren sind sehr vielfältig; neben den typischen Dünnschichttechniken werden auch Abformtechniken wie Lithographie, Galvanik und Abformung (LIGA), Ätztechniken usw. eingesetzt. Die Mikrosystemtechnik verwendet fast alle Arten von Materialien wie Metalle, Halbleiter, Keramiken, Sol-Gel-Materialien, Kunststoffe und viele mehr.

Die Mikrofluidik beschäftigt sich mit dem Verhalten von Flüssigkeiten und Gasen auf kleinstem Raum (Wikipedia, Stichwort Mikrofluidik).

Akustik ist die Lehre vom Schall (Wikipedia, Stichwort Akustik).

Akustische Oberflächenwellen (SAW) sind Festkörperwellen, die sich planar auf einer Oberfläche ausbreiten, d.h. nur in zwei Dimensionen (Wikipedia, Stichwort akustische Oberflächenwelle). Neben SAW gibt es weitere akustische Festkörperwellen, die auf Chipsubstraten angeregt werden können, z.B. akustische Plattenwellen, akustische Kantenwellen, akustische Grenzflächenwellen und akustische Volumenwellen.

Aerosole sind bekannt als heterogene Mischungen (Dispersionen) von festen und/oder flüssigen Schwebeteilchen in einem Gas (Wikipedia, Stichwort Aerosol). Aerosole, die nur flüssige Partikel in einem Gas enthalten, werden auch als Nebel oder Dunst bezeichnet.

Der Aerosolstrahldruck (engl. Aerosol Jet Printing,AJP) ist eine berührungslose Direktschreibtechnologie, die Flüssigkeiten mit hoher Präzision auf verschiedene Substrate aufträgt und das Drucken feiner elektronischer, struktureller und biologischer Muster ermöglicht. Der Aerosolstrahl kann auch als Aerosoljet bezeichnet werden.

Chemische oder physikalische Synthesesysteme sind bekannt als Systeme, die Gas- und/oder Aerosolströme in einer definierten Atmosphäre, z.B. einem Vakuum oder einer Inertgasatmosphäre, auf ein Substrat, z.B. einen Wafer, aufbringen, um ein Schicht- oder Strukturwachstum oder die Entfernung einer Schicht (Ätzen) zu erreichen. Zu den bekannten Methoden gehören die physikalische Gasphasenabscheidung (PVD, z.B. Sputtern oder Verdampfen), die chemische Gasphasenabscheidung (CVD), auch in Kombination mit Plasma-, Ionen- oder Mikrowellenquellen, die atomare Schichtabscheidung (ALD), das reaktive Ionenätzen (RIE) oder das atomare Schichtätzen (ALE).

Aufgrund der zunehmenden Komplexität technischer Systeme werden immer mehr Komponenten in elektronische Schaltungen integriert und für zahlreiche neue Anwendungen genutzt, insbesondere in der Mikrosystemtechnik. Insbesondere werden Komponenten aus der Mikrofluidik und/oder Komponenten, die akustische Oberflächenwellen erzeugen und/oder nutzen, in solche Systeme integriert, was zu einer kleineren Gerätegrundfläche, höherer Effizienz und deutlich breiteren und neuen Anwendungsbereichen führt. Ebenso werden Komponenten, die akustische Oberflächenwellen erzeugen oder nutzen, zunehmend in der Technik eingesetzt, die auch in Verbindung mit mikrofluidischen Komponenten verwendet werden können und dann als akustofluidische Komponenten bezeichnet werden.

Bekannte Anwendungsbereiche für die Mikrosystemtechnik mit Aerosolen sind insbesondere der Aerosolstrahldruck und die chemische Abscheidung (ALD, CVD), insbesondere unter Verwendung eines Transportgases mit einem chemischen Vorläufermaterial (Prekursor).

Beim Aerosolstrahldruck (AJP) Verfahren wird eine flüssige Phase, wie z.B. eine Metallpartikeldispersion, in Tröpfchen zerstäubt, das sogenannte Tintenaerosol, das dann durch eine Düse mit Hilfe eines fokussierenden Gasmantels auf eine Oberfläche übertragen wird. Der Aerosolstrahldruck ist ein direkt schreibendes Druckverfahren für die hochauflösende, maskenlose Materialabscheidung von Strukturen im Größenbereich von ca. 10 µm bis 5 mm. Bei diesem Verfahren wird das durch einen Aerosolgenerator erzeugte Aerosol mittels eines Gasstroms räumlich fokussiert und über eine Düse in einem Abstand im cm-Bereich auf eine zu beschichtende Oberfläche aufgebracht. Das AJP-Verfahren wird bereits in der Praxis im Bereich der gedruckten Elektronik eingesetzt, zum Beispiel bei der Herstellung von integrierten Antennen (Godlinski, D. et al: IET Microw. Antennas Propag. Article vol. 11, no. 14, pp 2010-2015, Nov. 2017), für das Chip-Packaging und für Hochfrequenzkontakte (https://optomec.com/applications/aerosol.jet), bei der Herstellung von Frontkontakten für Solarzellen (Binder, S.: Aerosol Jet Printing, Technische Universität Ilmenau 2017) und beim Druck von Leiterbahnen auf 3D-gedruckte Bauteile und Freiformkomponenten (http://investors.stratasys.com/news-events/pressreleases/detail/177/3d-printing-is-merged-with-printed Elektronik). Weitere Forschungsansätze befassen sich mit elektronischer Aufbau- und Verbindungstechnik (SMD, MID) (Krzeminski, J. et al: IEEE Trans. Nanotechnol. Article, Proceedings Paper vol. 17, no. 5, pp 979-984, Sept. 2018), mit der Leiterbahnintegration in Luft- und Raumfahrtstrukturen, mit dem Drucken von Fotodioden und dem Drucken von Piezosensoren. AJP wird auch zunehmend für den Druck biologischer Materialien eingesetzt.

Nach dem Stand der Technik wird die Aerosolerzeugung für den Aerosolstrahldruck derzeit mittels pneumatischer oder Ultraschallverfahren realisiert, die als separate Aerosolkammern ausgeführt sind. Anschließend wird das erzeugte Tintenaerosol von der Aerosolkammer mittels eines Trägergasstroms in den eigentlichen Druckkopf geleitet, wo es mittels eines koaxialen Fokussiergasstroms kollimiert, auf das zu beschichtende Substrat gerichtet und dort abgeschieden wird. Aufgrund der komplexen Systemarchitektur aktueller AJP-Aufbauten ist es nicht möglich, alle aerosol-, gas- und flüssigkeitstragenden Komponenten in einen kompakten Druckkopf zu integrieren, wodurch im Betrieb ein hoher Aufwand für das Einrichten und Reinigen der Aerosolkammer, der Versorgungsleitungen und des Druckkopfes entsteht. Bei kostenintensiven oder solchen Druckflüssigkeiten, die nur in kleinen Mengen verfügbar sind, stellt auch der Materialverlust in den Versorgungsleitungen oder durch große Start-/Totvolumina (mehrere ml) ein erhebliches Problem dar.

Bei der pneumatischen Zerstäubung für den Aerosolstrahldruck wird ein Trägergas über die Flüssigkeits-Gas-Grenzfläche beschleunigt, wodurch ein Bereich mit reduziertem statischen Druck entsteht, der die Flüssigkeit in den Trägergasstrom zieht. Die Instabilität der dünnen Flüssigkeitsschichten, die sich aus der Wechselwirkung mit dem umgebenden gasförmigen Medium ergibt, führt zu schnell wachsenden (Kapillar-)Wellen an der Flüssigkeits-Gas-Grenzfläche. Der Zerfall der Flüssigkeit tritt ein, wenn die Wellenamplitude einen kritischen Wert erreicht. Fragmente der Schichten werden abgerissen und ziehen sich unter der Wirkung der Oberflächenspannung schnell zu instabilen Bändern zusammen, und wenn diese Bänder kollabieren, bilden sich Tropfen.

Pneumatische Zerstäuber haben den Nachteil, dass sie mit hohem Druck über einen externen Drucklufterzeuger betrieben werden müssen und dass sie hohe Scherkräfte auf die Flüssigkeiten ausüben. Dies kann sensible Flüssigkeiten beschädigen und führt weiterhin zu einer breiten Tröpfchengrößenverteilung, die die Auflösung und Qualität der abgeschiedenen Strukturen verringert. Die sehr breite Tröpfchengrößenverteilung führt auch zu einer geringen Aerosolausbeute und damit zu einer niedrigeren Abscheiderate und erfordert den Einsatz eines virtuellen Impaktors/ Aerosolabscheiders zur Abscheidung größerer Aerosoltröpfchen und zur Druckreduzierung.

Weiterhin werden Ultraschall-Volumenwandler für die Erzeugung von Aerosolen für den Aerosolstrahldruck eingesetzt. Bei diesen Geräten wird eine Flüssigkeit durch einen Ultraschallwandler akustisch angeregt. Im Reservoir werden (kapillare) Wellen an der Flüssigkeits-Gas-Grenzfläche erzeugt und bewirken, dass die Flüssigkeit in ein Aerosol aus Tröpfchen unterschiedlicher Größe zerfällt, das dann mit einem Transportgasstrom entfernt wird. Diese Lösung hat jedoch einige erhebliche Nachteile, wie ein großes, unhandliches Gerät, ein hohes Totvolumen und damit Probenverluste sowie einen sehr hohen Reinigungsaufwand. Ultraschallzerstäuber sind nur für Flüssigkeiten mit einer relativ niedrigen Viskosität von 1-20 mPas geeignet. Dies schränkt die Materialauswahl für die Flüssigkeiten ein, da insbesondere viele biologische Flüssigkeiten eine höhere Viskosität aufweisen und auf diese Weise überhaupt nicht zerstäubt werden können.

Für ein homogenes Druckbild und einen kontinuierlichen Druck sollte der Flüssigkeitsstand in der Aerosolkammer auch bei der ultraschallbasierten Aerosolerzeugung konstant gehalten werden, was allerdings eine sehr komplexe Nachfüllkonstruktion erfordert.

Die kontinuierliche Rezirkulation der Tintenaerosole in der Aerosolkammer führt bei beiden Technologien (pneumatisch und Ultraschall) zum Verdampfen der leicht flüchtigen Lösungsmittel in den Flüssigkeiten und verändert deren Zusammensetzung, was sich ebenfalls negativ auf die Druckqualität auswirkt und die Aerosolausbeute verringert.

Neben den oben genannten Nachteilen einzelner Lösungen haben die bekannten Lösungen aus dem Stand der Technik den Nachteil, dass die Geräte und Komponenten zur Erzeugung von Aerosolen in ihren Abmessungen zu groß sind und daher nicht uneingeschränkt in andere Geräte integriert werden können und selbst nicht in ihren Abmessungen reduziert und miniaturisiert werden können. Ebenso können verschiedene Eigenschaften des Aerosols, wie die Geometrie oder die Geschwindigkeit des Aerosolstrahls und die Temperatur der Flüssigkeit, noch nicht in weiten Grenzen präzise genug eingestellt werden.

Lösungen für die SAW-basierte Aerosolerzeugung sind bereits bekannt (Roudini, M. et al: J of Aerosol Science, Juli 18, 2023). Die SAW-Aerosolerzeugungschips verfügen über fokussierte und gerade Interdigitalwandler (IDTs) und integrierte mikrofluidische Flüssigkeitszufuhrkanäle, die im Wafermaßstab durch mehrstufige Trockenfilm-Fotolack-Laminierung (Dry-film Lamination) und lithografische Strukturierungstechniken hergestellt werden.

Die Aufgabe der vorliegenden Erfindung besteht darin, eine akustofluidische Vorrichtung zur Erzeugung von Aerosolen, d.h. eine Aerosolgeneratorbaugruppe, bereitzustellen, die geringe Abmessungen aufweist und in Geräte oder Systeme zur Abscheidung von Aerosolen integriert werden kann und durch einen einfachen Aufbau einen gerichteten Aerosolstrom mit einstellbaren Aerosoleigenschaften realisiert.

Die Aufgabe wird durch die in den Ansprüchen genannte Erfindung gelöst. Vorteilhafte Ausführungsformen sind Gegenstand der Unteransprüche, wobei die Erfindung auch Kombinationen der einzelnen abhängigen Ansprüche im Sinne einer Und-Kombination umfasst, sofern sie sich nicht gegenseitig ausschließen.

Die erfindungsgemäße akustofluidische Vorrichtung zur Erzeugung von Aerosolen enthält mindestens
- mindestens eine Aerosolauslasskomponente mit mindestens einer Öffnung, aus der ein auf gerichteter Aerosolstrahl oder -jet austritt, und
- mindestens eine Halterkomponente für mindestens eine mikroakustische Komponente, und
- mindestens je eine Zu- und/oder Ableitung für Gase, Flüssigkeiten und Wärme, mindestens eine elektrische Leitung und/oder elektrische Signalleitung und mindestens eine elektrische Hochfrequenzsignalleitung, und
- mindestens eine Komponente zum Wärmetransport, die den Wärmetransport von und/oder zu mindestens einer Halterkomponente realisiert, und die mit mindestens einer Zu- und/oder Ableitung für Wärme verbunden ist, und
- mindestens einen Temperatursensor, der thermisch leitend mit der Halterkomponente und/oder der Wärmetransportkomponente und elektrisch leitend mit mindestens einer elektrischen Hochfrequenzsignalleitung verbunden ist, und
- mindestens eine mikroakustische Komponente mit einer lateralen Abmessung von maximal 4 × 4 cm² mit mindestens einem Schallwandler und/oder elektroakustischen und/oder piezoelektrischen Wandler für die Anregung akustischer Wellen im Frequenzbereich von 1 bis 500 MHz, und
- mindestens eine Komponente zur Übertragung eines Hochfrequenzsignals, die elektrisch leitend und hochfrequenztauglich mit mindestens einer elektrischen Hochfrequenzsignalleitung und mit mindestens einer mikroakustischen Komponente verbunden ist, und die die Übertragung eines Hochfrequenzsignals über die mindestens eine elektrische Hochfrequenzsignalleitung zu und/oder von der mindestens einen mikroakustischen Komponente realisiert, und
- mindestens eine Komponente für den Flüssigkeitstransport, die mit mindestens einer Zu- und/oder Ableitung für Flüssigkeiten und mit mindestens der einen mikroakustischen Komponente flüssigkeitsführend verbunden ist, und mit der mindestens die Zuführung von Flüssigkeiten mit einem Volumenstrom zwischen 0,1 µl/min und 5 ml/min auf die Oberfläche der mindestens einen mikroakustischen Komponente realisiert ist, und
- mindestens eine Transportgaskomponente, die mit mindestens einer Zu- und/oder Ableitung für Gase verbunden ist, wobei mit der Transportgaskomponente mindestens die Zufuhr mindestens eines Gases in die unmittelbarer Nähe des Bereichs der akustofluidischen Wechselwirkung zwischen der akustischen Welle und der Flüssigkeit zur Aerosolerzeugung auf der flüssigkeitsbeaufschlagten Oberfläche der mikroakustischen Komponente realisiert ist, und
- mindestens eine Komponente zur Positionierung und/oder mechanischen Fixierung der mindestens einen Komponente zur Übertragung eines Hochfrequenzsignals und der mindestens einen Komponente für den Flüssigkeitstransport in Bezug zu der mindestens einen mikroakustischen Komponente oder zu der mindestens einen Halterkomponente, wobei die mindestens eine Komponente zur Positionierung und/oder mechanischen Fixierung eine unabhängige Komponente und/oder eine Komponenten der Halterkomponente und/oder der Komponente für den Flüssigkeitstransport und/oder der Aerosolauslasskomponente und/oder einer anderen Komponente ist.

Vorteilhafterweise realisiert die mindestens eine mikroakustische Komponente die Anregung mindestens einer akustischen Welle, vorteilhafterweise einer akustischen Rayleigh-Oberflächenwelle und/oder einer akustischen Sezawa-Oberflächenwelle, und/oder einer akustischen Plattenwelle, vorteilhafterweise einer symmetrischen und/oder asymmetrischen Lamb-Welle, und/oder einer akustischen Volumenwelle, vorteilhafterweise einer Longitudinal- und/oder Scherwelle, und/oder einer stehenden akustischen Oberflächenwelle und/oder einer Körper- und/oder Flüssigkeitsschallwelle.

Auch vorteilhafterweise ist die mindestens eine Komponente für den Flüssigkeitstransport mit mindestens einer Flüssigkeitspumpe und/oder mindestens einem Flüssigkeitsreservoir verbunden oder beinhaltet mindestens eine Flüssigkeitspumpe und/oder mindestens ein Flüssigkeitsreservoir und/oder mindestens einer Mikropumpe und/oder mindestens einer elektroosmotischen Pumpe und/oder mindestens einem Flüssigkeitsventil und/oder mindestens einer sensorischen und/oder analytischen Komponente.

Weiterhin vorteilhafterweise realisiert mindestens eine Komponente zur Positionierung und/oder mechanischen Fixierung ein reversibles vertikales Kippen im Sinne einer Teildrehung oder ein reversibles vertikales Anheben im Sinne einer Verschiebung oder ein reversibles Gleiten mindestens der mindestens einen Komponente zur Übertragung eines Hochfrequenzsignals und der mindestens einen Komponente für den Flüssigkeitstransport in Bezug zu der mindestens eine mikroakustische Komponente oder zu der mindestens einen Halterkomponente zum Austausch der mikroakustischen Komponente, wobei noch vorteilhafterweise die reversible Installation der mindestens einen mikroakustischen Komponente durch seitliches Gleiten, Einsetzen oder Verschieben der mikroakustischen Komponente in oder auf die Halterkomponente oder durch vertikales Auflegen oder Einsetzen der mikroakustischen Komponente in oder auf die Halterkomponente realisiert ist.

Ebenfalls vorteilhafterweise enthält die mindestens eine Wärmetransportkomponente mindestens ein Wärmerohr und/oder einen Kühlkörper mit oder ohne Komponenten zur Verbesserung der Gaskonvektion und/oder mit damit verbundener Flüssigkeitskühlung und/oder mindestens ein Peltier-Element mit einem passiven Rippenkühler mit oder ohne Komponenten zur Verbesserung der Gaskonvektion und/oder mindestens ein Peltier-Element mit einem Kühlkörper mit damit verbundener Flüssigkeitskühlung oder ist damit verbunden.

Es ist auch vorteilhaft, wenn die mindestens eine mikroakustische Komponente ein mikroakustischer Chip oder eine mikroakustische Kartusche ist, wobei die mikroakustische Kartusche mindestens aus mindestens einem mikroakustischen Chip auf mindestens einer Platte oder Folie aus einem Material mit hoher Wärmeleitfähigkeit besteht, und wobei zusätzliche Schichten aus hochwärmeleitfähigem Material mindestens zwischen Chip und Platte oder Folie und/oder auf der dem Chip abgewandten Seite der Platte oder Folie vorhanden sind, wobei noch vorteilhafterweise eine hochwärmeleitende Verbindung zwischen dem mikroakustischen Chip und der Platte oder Folie mit einem Klebstoff und/oder einer haftungsfördernden Flüssigkeit und/oder ein- oder doppelseitigem Klebeband und/oder metall- und/oder keramikgefüllten Polymeren, Epoxiden und/oder Pasten und/oder einem Klebeband oder Klebestreifen und/oder mittels eines nichtpermanenten Klebstoffs realisiert ist.

Weiterhin vorteilhaft ist es, wenn mindestens eine mikroakustische Komponente und/oder mindestens eine Komponente für den Flüssigkeitstransport mindestens ein mikrofluidisches und/oder flüssigkeitsleitendes Strukturelement aufweist und die mindestens eine Komponente für den Flüssigkeitstransport mit der mikroakustischen Komponente über mindestens ein mikrofluidisches und/oder flüssigkeitsleitendes Strukturelement flüssigkeitsleitend verbunden ist, wobei noch vorteilhafterweise mindestens ein Mikrokanal und/oder ein Verbindungselement und/oder ein Dichtungselement und/oder ein Reservoir und/oder eine Mikromembran und/oder ein nadelförmiges Strukturelement und/oder ein schwamm- oder gewebeförmiges Element als mikrofluidisches und/oder flüssigkeitsleitendes Strukturelement vorhanden ist.

Ebenfalls ist es vorteilhaft, wenn mindestens eine Transportgaskomponente ein koaxiales Einströmen eines Gases in den Bereich der akustofluidischen Wechselwirkung zwischen der akustischen Welle und der Flüssigkeit zur Umhüllung des erzeugten Aerosolstrahls oder -jets realisiert, wobei die Stelle des Austritts des Gases aus der Transportgaskomponente höchstens 30 mm, vorteilhafterweise < 15 mm, noch vorteilhafter < 5 mm, entfernt angeordnet ist von der flüssigkeitstragenden Oberfläche der mindestens einen mikroakustischen Komponente in Richtung des Volumenstromvektors des Aerosolstrahls oder -jets, wobei noch vorteilhafterweise der rotationssymmetrische Volumenstromvektor des koaxial einströmenden Gases um nicht mehr als 45°, vorteilhafterweise um nicht mehr als 30°, vom Volumenstromvektor des Aerosolstrahls oder -jets, oder von der Oberflächennormalen der flüssigkeitstragenden Oberfläche der mindestens einen mikroakustischen Komponente abweicht.

Und auch vorteilhaft ist es, wenn stromabwärts von der mindestens einen Transportgaskomponente oder nach der mindestens einen Öffnung in der Aerosolaustrittskomponente in Richtung des Volumenstromvektors des Aerosolstrahls oder -jets mindestens eine Komponente zur aerodynamischen Fokussierung des Aerosolstrahls oder -jets mit einem Gasmantel und/oder zur Erhöhung der Geschwindigkeit des Aerosolstrahls oder -jets vorhanden ist, die mindestens eine weitere Zu- und/oder Ableitung für Gase aufweist.

Von Vorteil ist es weiterhin, wenn ein zusätzliches rohrförmiges Gehäuse die akustofluidische Vorrichtung umschließt, welches aus Metall, Polymer, Keramik oder Glas besteht, und welches an mindestens einem Rohrende mindestens einen Flansch der Bauart ISO-K, ISO-KF, ISO-F, CF oder COF für die Zu- und Ableitungen und elektrische Signalleitungen und elektrische Hochfrequenzsignalleitungen aufweist.

Ebenfalls von Vorteil ist es, wenn die Öffnung oder die Öffnungen in der Aerosolauslasskomponente Abmessungen von nicht größer als 5 mm aufweisen und/oder eine kreisförmige, koaxiale und/oder ovale Form haben und/oder Mehrfachöffnungsauslässe sind und/oder Luer- und/oder metrische und/oder zöllige Gewinde aufweisen.

Vorteilhaft ist es auch, wenn mindestens eine Steuer- und/oder Regelkomponente zur Steuerung und/oder Regelung des Austritts des Aerosolstrahls oder -jets aus der Aerosolaustrittskomponente oder aus der Komponente zur aerodynamischen Fokussierung des Aerosolstrahls oder -jets vorhanden ist, die an der Außenseite oder Innenseite der Aerosolaustrittskomponente oder der Komponente zur aerodynamischen Fokussierung des Aerosolstrahls oder -jets oder stromabwärts oberhalb der mikroakustischen Komponente angeordnet ist und/oder Teil der Komponente für den Flüssigkeitstransport ist, wobei noch vorteilhafterweise die Steuer- und/oder Regelkomponente ein drehbarer oder beweglicher Balken ist, der im Aerosolstrahl oder -jet positioniert ist und/oder eine Komponente zum Verschließen der Öffnung oder der Öffnungen ist und/oder eine Komponente ist, die eine Absaugvorrichtung enthält.

Die vorliegende Erfindung ermöglicht es erstmals, eine akustofluidische Vorrichtung zur Erzeugung von Aerosolen, zu spezifizieren, die geringe Abmessungen aufweist und die in Geräte oder Systeme zur Abscheidung von Aerosolen integriert werden kann und die durch einen einfachen Aufbau einen gerichteten Aerosolstrom mit einstellbaren Aerosoleigenschaften realisiert.

Dies wird durch eine akustofluidische Vorrichtung zur Erzeugung von Aerosolen erreicht, bei der die Aerosolerzeugung durch die Wechselwirkung der zu zerstäubenden Flüssigkeit mit einer hochfrequenten akustischen Welle erreicht wird, die von mindestens einer mikroakustischen Komponente erzeugt wird. Die Zerstäubung mittels einer hochfrequenten akustischen Welle führt zu Aerosolen mit sehr kleinen Tröpfchen und einer sehr homogenen Tröpfchengrößenverteilung, die reproduzierbar und dynamisch in Bezug auf die Tröpfchengröße eingestellt werden kann. Mit der mindestens einen mikroakustischen Komponente kann auch ein breiter Parameterbereich von Flussraten realisiert werden. Außerdem können die Eigenschaften des Aerosolstrahls (Geschwindigkeit, Breite und Winkel) angepasst werden, was eine effizientere Fokussierung des Aerosolstrahls ermöglicht.

Die erfindungsgemäße akustofluidische Vorrichtung zur Erzeugung von Aerosolen enthält mindestens eine Aerosolauslasskomponente, mindestens eine Halterkomponente, mindestens jeweils Zu- und/oder Ableitungen für Gase, Flüssigkeiten und Wärme, mindestens ein Wärmetransportkomponente, mindestens einen Temperatursensor, mindestens eine mikroakustische Komponente, mindestens eine Komponente zur Übertragung eines Hochfrequenzsignals, mindestens eine Komponente für den Flüssigkeitstransport, mindestens eine Transportgaskomponente und mindestens eine Komponente zur Positionierung und/oder mechanischen Fixierung.

Die erfindungsgemäße mindestens eine Aerosolauslasskomponente gemäß der vorliegenden Erfindung hat mindestens eine Öffnung, aus der ein gerichteter Aerosolstrahl oder -jet austritt.

Es ist vorteilhaft, wenn die mindestens eine Öffnung in der Aerosolauslasskomponente, aus der ein gerichteter Aerosolstrahl oder -jet austritt, Abmessungen nicht größer als 5 mm aufweist und/oder eine kreisförmige, koaxiale und/oder ovale Form aufweist und/oder Mehrfachöffnungsauslässe sind und/oder Luer- und/oder metrische und/oder zöllige Gewinde aufweisen.

Erfindungsgemäß gibt es mindestens je eine Zu- und/oder Ableitung für Gase, Flüssigkeiten und Wärme, sowie mindestens eine elektrische Leitung und/oder elektrische Signalleitung und mindestens eine elektrische Hochfrequenzsignalleitung.

Für die Integration in übergeordnete Systeme, z.B. chemische oder physikalische Synthesesysteme, ist es von Vorteil, wenn die erfindungsgemäße akustofluidische Vorrichtung in ein rohrförmiges Gehäuse eingebaut vorliegt, welches aus Metall, Polymer, Keramik oder Glas bestehen kann und welches an mindestens einem Rohrende mindestens ein Flansch, z.B. der Bauart ISO-K, ISO-KF, ISO-F, CF oder COF für die Zu- und/oder Ableitungen und elektrische Signalleitungen und elektrische Hochfrequenzsignalleitungen aufweist.

Weiter ist erfindungsgemäß mindestens eine Halterkomponente für mindestens eine mikroakustische Komponente vorhanden.

Darüber hinaus verfügt die erfindungsgemäße akustofluidische Vorrichtung über mindestens eine Wärmetransportkomponente, die den Wärmetransport von und/oder zu der mindestens einen Halterkomponente realisiert. Die Wärmetransportkomponente ist mit mindestens einer Zu- und/oder Abfuhrleitung für Wärme verbunden.

Vorteilhaft ist, dass die Halterkomponente zumindest teilweise aus einem Material mit hoher Wärmeleitfähigkeit besteht. Zwischen der Wärmetransportkomponente und der Halterkomponente können sich auch eine oder mehrere zusätzliche Schichten aus temperaturleitfähigem Material befinden.

Für die Komponenten der erfindungsgemäßen akustofluidischen Vorrichtung können eine Vielzahl von Materialien verwendet werden, z.B. metallische oder nichtmetallische Materialien, Polymere, insbesondere druckbare oder CNC-fräsbare Polymere oder polymerhaltige Komposit-Materialien, Gläser, Oxide, Keramiken oder Verbundwerkstoffe. Die Komponenten können jeweils aus einem einzigen Material bestehen oder aus mehreren verschiedenen Materialien und/oder Komponenten zusammengesetzt sein. Ebenfalls ist die Vereinigung / Zusammenführung von einzelnen Komponenten der erfindungsgemäßen akustofluidischen Vorrichtung in einer kombinierten Komponente möglich, wie beispielsweise die Kombination der Aerosolauslasskomponente und der Transportgaskomponente.

Die erfindungsgemäße mindestens eine Wärmetransportkomponente verhindert Temperaturschwankungen der Halterkomponente und Temperaturabweichungen von einem Sollwert der Halterkomponente und damit der Flüssigkeiten, die zu einem Aerosol zerstäubt werden sollen. Damit können konstante Bedingungen bei der Aerosolerzeugung gewährleistet werden und Temperaturregime an die Erfordernisse der Flüssigkeiten und/oder des Druckprozesses angepasst werden.

Die mindestens eine Wärmetransportkomponente kann mindestens Folgendes umfassen ein Wärmerohr (Heat pipe) und/oder einen Kühlkörper mit oder ohne Komponenten zur Verbesserung der Gaskonvektion und/oder der damit verbundenen Flüssigkeitskühlung, und/oder mindestens ein Peltier-Element mit einem passiven Rippenkühler mit oder ohne Komponenten zur Verbesserung der Gaskonvektion und/oder mindestens ein Peltier-Element mit einem Kühlkörper mit angeschlossener Flüssigkeitskühlung.

Ein Peltier-Element ist ein elektrothermischer Wandler, der mit Hilfe eines Stromflusses einen Temperaturunterschied auf der Grundlage des Peltier-Effekts erzeugt. Peltier-Elemente können sowohl zum Kühlen als auch zum Heizen verwendet werden (Wikipedia, Stichwort Peltier-Element).

Wenn die Flüssigkeitskühlung ein Teil der Wärmetransportkomponente ist, können dies flexible oder starre Rohre, Rohre, Kanäle oder andere flüssigkeitsführende Elemente sein. Durch sie kann die Temperatur der Wärmetransportkomponente und/oder des Peltier-Elements, und damit die Temperatur der Halterkomponente verändert und gesteuert werden und insbesondere die Temperatur der Halterkomponente oder des Peltier-Elements auf einen Sollwert erhöht oder gesenkt werden.

Erfindungsgemäß ist mindestens ein Temperatursensor zur Erfassung der Temperatur der Halterkomponente und/oder der Temperatur der Wärmetransportkomponente vorgesehen, der thermisch leitend mit der Halterkomponente und/oder der Wärmetransportkomponente und elektrisch leitend mit mindestens einer elektrischen Signalleitung verbunden ist.

Erfindungsgemäß gibt es mindestens eine mikroakustische Komponente mit einer lateralen Abmessung von maximal 4 × 4 cm² mit mindestens einem Schallwandler und/oder elektroakustischen und/oder piezoelektrischen Wandler für die Anregung akustischer Wellen im Frequenzbereich von 1 bis 500 MHz.

Der Begriff Mikroakustik wird hier verwendet, um Systeme zu beschreiben, die auf der Anregung, Ausbreitung und/oder Interaktion von akustischen Wellen mit einer typischen Wellenlänge von weniger als einem Millimeter basieren. Das bedeutet, dass mikroakustische Systeme auf Arbeitsfrequenzen oberhalb des hörbaren Spektrums beruhen.

Die mindestens eine mikroakustische Komponente weist erfindungsgemäß einen Oberflächenbereich auf, in dem eine Wechselwirkung zwischen der akustischen Welle und dem Fluid zur Aerosolerzeugung realisiert wird.

Akustische Oberflächenwellen, vorteilhaft akustische Rayleigh-Oberflächenwellen und/oder akustische Sezawa-Oberflächenwellen, und/oder akustische Plattenwellen, vorteilhaft symmetrische und/oder asymmetrische Lamb-Wellen, und/oder akustische Volumenwellen, vorteilhaft Longitudinal- und/oder Scherwellen, und/oder stehende akustische Oberflächenwellen und/oder Körper- und/oder Flüssigkeitsschallwellen können in der mikroakustischen Komponente als akustische Wellen angeregt werden. Noch vorteilhafterweise können stehende akustische Rayleigh-Oberflächenwellen angeregt werden.

Vorteilhafterweise kann die mindestens eine mikroakustische Komponente ein mikroakustischer Chip oder eine mikroakustische Kartusche (Cartridge) sein, wobei die mikroakustische Kartusche mindestens aus mindestens einem mikroakustischen Chip auf mindestens einer Platte oder Folie aus einem Material mit hoher Wärmeleitfähigkeit besteht. Die Platte oder Folie, auf der der mikroakustische Chip befestigt ist, kann z.B. aus Metall, Polymer, Glas, Keramik oder einem Kompositmaterial hergestellt sein und/oder auch aus einer mehrlagigen Platte bestehen. Die Platte oder Folie kann auch Führungselemente für die Befestigung an der Halterkomponente aufweisen. Ebenfalls kann die Platte oder Folie an der Seite, an der sie in der akustofluidischen Vorrichtung in Kontakt mit der Halterkomponente steht, eine wärmeleitfähige Schicht aufweisen.

Die mikroakustische Komponente und insbesondere ein mikroakustischer Chip verfügen über mindestens einen Schallwandler und/oder elektroakustischen und/oder piezoelektrischen Wandler, der elektrisch mit mindestens einer Komponente zur Übertragung eines Hochfrequenzsignalleitung verbunden ist.

Vorteilhafterweise ist der Wandler der mikroakustischen Komponente ein Interdigitalwandler aus einem strukturierten Metall-Dünnfilm.

Die mikroakustische Komponente und insbesondere ein mikroakustischer Chip können vorteilhafterweise ein piezoelektrisches Material und/oder mindestens eine Schicht aus einem piezoelektrischen Material auf einem nicht-piezoelektrischen Material und/oder mindestens ein nicht-piezoelektrisches Material auf einem piezoelektrischen Material aufweisen. Vorteilhaft sind als Materialien für die mikroakustische Komponente nicht-piezoelektrische Materialien wie Glas, Keramik, Glas/Keramik-Verbundwerkstoffe, Verbundwerkstoffe mit SiO₂, Al₂O₃, Si₃N₄, TiN, SiN oder Borosilikatglas, oder wie Metalle/Metalllegierungen, wie Al, Cu, Ti, Ta, TiAl, CuTi, oder Polymere, wie PMMA, PTFE, PEEK, Polyimid, PET, COP, PDMS, PC, COC, Polycaprolacton, PS, oder Photoresists, wie SUEX, ADEX, TMMF S2045, Ordyl, SU-8, oder Halbleitermaterialien, wie Si, GaAs, InAs, GaN und/oder piezoelektrische Materialien, wie Quarz, LiNbO₃, schwarzes LiNbO₃, gelb-schwarzes LiNbO₃, LiTaO₃, AlN, Sc-AlN, ZnO, TaGa₃, CTGS, SiO₂, Langasit, Galliumorthophosphat, PZT, PMN-PT oder PVDF oder Kombinationen dieser Materialien vorhanden.

Darüber hinaus können zwischen dem mikroakustischen Chip und der Platte oder der Folie einer mikroakustischen Kartusche sowie zwischen der Platte oder Folie einer mikroakustischen Kartusche und der Halterkomponente vorteilhaft eine oder mehrere zusätzliche Schichten aus einem hochwärmeleitende Material angeordnet sein, die aus Klebstoff und/oder haftungsfördernder Flüssigkeit bestehen und/oder ein- oder doppelseitigem Klebeband, metall- und/oder keramikgefüllte Polymere, Epoxide und/oder Pasten und/oder ein Klebeband oder Klebestreifen und/oder ein nichtpermanenter Klebstoff sein können. Beispielmaterialien dafür sind Silikone mit Graphit- oder Keramikpartikeln und mit gewebten Kohlenstofffasern oder anderen Geweben, sowie silikonfreie weiche Polymere mit den oben genannten Füllstoffen oder Geweben oder Lückenfüller-Materialien (Gap-Filler).

Weiterhin ist erfindungsgemäß mindestens eine Komponente für den Flüssigkeitstransport vorgesehen, die mit mindestens einer Zu- und/oder Ableitung für Flüssigkeiten und mit der mindestens einen mikroakustischen Komponente flüssigkeitsführend verbunden ist, und mit der mindestens die Zuführung von Flüssigkeiten mit einem Volumenstrom zwischen 0,1 µl/min und 5 ml/min auf die Oberfläche der mindestens einen mikroakustischen Komponente, auf der die Interaktion der Flüssigkeit mit der akustischen Welle erfolgt, realisiert wird.

Vorteilhafterweise ist die mindestens eine Komponente für den Flüssigkeitstransport mit mindestens einer Flüssigkeitspumpe und/oder mindestens einem Flüssigkeitsreservoir verbunden oder mindestens einer Flüssigkeitspumpe und/oder mindestens einem Flüssigkeitsreservoir und/oder mindestens eine Mikropumpe und/oder mindestens eine elektroosmotische Pumpe und/oder mindestens ein Flüssigkeitsventil und/oder mindestens eine sensorische und/oder analytische Komponente beinhaltet.

Die Komponente für den Flüssigkeitstransport kann vollständig oder teilweise aus einem Polymer, wie Polydimethylsiloxan, Polystyrol, Polycaprolacton, Polycarbonat, PEEK, PTFE, PP, PE, Cyclo-Olefin-Copolymer, Polymethylmethacrylat, Polyimid, Polyetheretherketon, aus Glas, Silizium, Metall, Keramik und/oder aus photolithographisch strukturierten Polymeren, wie SU-8, DF3500, ADEX, SUEX oder KMPR-Photoresist, bestehen.

Weiterhin ist es von Vorteil, wenn die mindestens eine mikroakustische Komponente und/oder die mindestens eine Komponente für den Flüssigkeitstransport mindestens ein mikrofluidisches und/oder flüssigkeitsführendes Strukturelement aufweist und die mindestens eine Komponente für den Flüssigkeitstransport flüssigkeitsleitende mit der mindestens einen mikroakustischen Komponente über mindestens ein flüssigkeitsleitendes Strukturelement verbunden ist.

Vorteilhafterweise können mindestens ein Mikrokanal und/oder ein Verbindungselement und/oder ein Dichtelement und/oder ein Reservoir und/oder eine Mikromembrane und/oder ein nadelförmiges Strukturelemente und/oder ein schwamm- oder gewebeförmiges Elemente als mikrofluidisches und/oder flüssigkeitsleitendes Strukturelement vorhanden sein.

Weiterhin ist erfindungsgemäß mindestens ein Bereich auf der Oberfläche der mindestens einen mikroakustischen Komponente vorhanden, auf der eine akustofluidische Wechselwirkung zwischen der akustischen Welle und der Flüssigkeit zur Aerosolerzeugung realisiert ist.

Gemäß der erfindungsgemäßen Lösung soll die Flüssigkeit, die in ein Aerosol umgewandelt wird, mittels des mindestens einen mikrofluidischen und/oder flüssigkeitsführenden Strukturelements der mikroakustischen Komponente und/oder der Komponente für den Flüssigkeitstransport zumindest teilweise in den Bereich auf der Oberfläche der mikroakustische Komponente geleitet werden, in dem die akustofluidische Wechselwirkung zwischen akustischer Welle und Flüssigkeit realisiert ist.

Das mindestens eine mikrofluidische und/oder flüssigkeitsführende Strukturelement oder Strukturelemente können zumindest teilweise außerhalb des Oberflächenbereiches der mikroakustischen Komponente angeordnet sein, wo die akustofluidische Wechselwirkung oder Interaktion zwischen der akustischen Welle und der Flüssigkeit stattfindet.

Erfindungsgemäß muss jedoch mindestens eine Öffnung der Komponente für den Flüssigkeitstransport oder des mindestens einen mikrofluidischen und/oder flüssigkeitsführenden Strukturelements, aus denen die Flüssigkeit austritt, im Bereich oder in der Nähe des Oberflächenbereichs der mikroakustischen Komponente angeordnet sein, wo die akustofluidischen Wechselwirkung zwischen der akustischen Welle und der Flüssigkeit stattfindet. Vorteilhafterweise ist diese Öffnung der Komponente für den Flüssigkeitstransport oder des mindestens einen mikrofluidischen und/oder flüssigkeitsführenden Strukturelements an dem Punkt oder dem Bereich angeordnet, an dem eine nachweisbare Zunahme der Amplitude der angeregten Welle auftritt. Dieser nachweisbare Anstieg der Amplitude kann vorteilhaft 5-30 %, noch vorteilhafter 5-15 % der maximalen Amplitude der Welle oder Wellen oder 10⁻⁴ bis 10 nm/mm, vorteilhaft 10⁻³ bis 5 nm/mm, betragen.

Bei den Flüssigkeiten, die in ein Aerosol umgewandelt werden sollen, kann es sich um technische Tinten, leitfähige oder nichtleitfähige Flüssigkeiten mit oder ohne Nanopartikel, partikelfreie Flüssigkeiten, Flüssigkeiten mit Metallpartikeln, Flüssigkeiten, die biologische Bestandteile, wie z.B. Moleküle, DNA, RNA, Proteine, Enzyme, Lypopolymere, Zucker, Antikörper, Vesikel, Zellen und andere biologische Bestandteile (Bioflüssigkeiten, Bioinks) enthalten, oder um reines Glycerin, Flüssigkeiten auf Harzbasis, technische Flüssigkeiten, geschmolzene Metalle oder Polymere oder Graphittinte handeln.

Erfindungsgemäß ist ferner mindestens eine Komponente zur Übertragung eines Hochfrequenzsignals vorhanden, die elektrisch leitend und hochfrequenztauglich mit mindestens einer elektrischen Hochfrequenzsignalleitung und mit der mindestens einen mikroakustischen Komponente verbunden ist und die die Übertragung eines Hochfrequenzsignals über die mindestens eine elektrische Hochfrequenzsignalleitung zu und/oder von der mindestens einen mikroakustischen Komponente realisiert.

Eine solche Komponente zur Übertragung eines Hochfrequenzsignals kann vorteilhaft eine Leiterplatte, eine flexible Leiterplatte oder ein geschichtetes Bauteil mit integrierten elektrischen Leitungen sein. Die elektrisch leitende Verbindung zur mikroakustischen Komponente kann vorteilhaft über Federstifte, vorzugsweise über hochfrequenzgeeignete, impedanzangepasste Federstifte, Federn und/oder über kapazitive oder induktive Signalkopplung hergestellt werden.

Weiterhin ist erfindungsgemäß mindestens eine Transportgaskomponente vorgesehen.

Unter einer Transportgaskomponente soll im Rahmen der vorliegenden Erfindung ein Bauteil verstanden werden, mit dem der Transport eines Gases mindestens in den Bereich der akustofluidische Wechselwirkung zwischen akustischer Welle und Flüssigkeit auf der Oberfläche des mikroakustischen Komponente realisiert ist.

Die Transportgaskomponente ist mit mindestens einer Zu- und/oder Ableitung für Gase verbunden, wobei mit der Transportgaskomponente mindestens die Zufuhr mindestens eines Gases in die unmittelbare Nähe des Bereichs der akustofluidischen Wechselwirkung zwischen der akustischen Welle und der Flüssigkeit zur Aerosolerzeugung auf der flüssigkeitsbeaufschlagten Oberfläche der mikroakustischen Komponente realisiert wird.

Vorteilhafterweise realisiert die mindestens eine Transportgaskomponente das koaxiale Einströmen eines Gases in den Bereich der akustofluidischen Wechselwirkung zwischen der akustischen Welle und der Flüssigkeit zur Umhüllung des erzeugten Aerosolstrahls oder -jets.

Vorteilhafterweise ist die Stelle des Austritts des Gases aus der Transportgaskomponente höchstens 30 mm, vorteilhafterweise < 15 mm, noch vorteilhafter < 5 mm, entfernt angeordnet von der flüssigkeitstragenden Oberfläche der mindestens einen mikroakustischen Komponente in Richtung des Volumenstromvektors des Aerosolstrahls oder -jets

Außerdem ist es vorteilhaft, wenn der rotationssymmetrische Volumenstromvektor des koaxial einströmenden Gases um nicht mehr als 45°, vorteilhafterweise um nicht mehr als 30°, vom Volumenstromvektor des Aerosolstrahls oder -jets, oder von der Oberflächennormalen der flüssigkeitsführenden Oberfläche der mindestens einen mikroakustischen Komponente abweicht.

Vorteilhaft ist es auch, wenn stromabwärts von der mindestens einen Transportgaskomponente oder nach der mindestens einen Öffnung in der Aerosolaustrittskomponente in Richtung des Volumenstromvektors des Aerosolstrahls oder -jets mindestens eine weitere Komponente zur aerodynamischen Fokussierung mit einem Gasmantel zu einem Aerosolstrahl oder -jet, und/oder zur Erhöhung der Geschwindigkeit des Aerostrahls oder -jets vorhanden ist, die mindestens eine weitere Zu- und/oder Ableitung für Gase aufweist.

Vorteilhafterweise ist es, wenn die weitere Komponente zur Fokussierung und Geschwindigkeitserhöhung nachgeschaltet ist und beispielsweise ein Filter, eine Düse oder einen Trichter ist oder enthält.

Erfindungsgemäß ist ferner mindestens eine Komponente zur Positionierung und/oder mechanischen Fixierung der mindestens einen Komponente zur Übertragung eines Hochfrequenzsignals und der mindestens einen Komponente für den Flüssigkeitstransport in Bezug zu der mindestens einen mikroakustischen Komponente oder zu der mindestens einem Halterkomponente vorhanden.

Dabei kann die mindestens eine Komponente zur Positionierung und/oder mechanischen Fixierung erfindungsgemäß eine unabhängige Komponente und/oder eine Komponente der Halterkomponente und/oder der Komponente für den Flüssigkeitstransport und/oder der Aerosolaustrittskomponente und/oder einer anderen Komponente sein.

Vorteilhafterweise realisiert mindestens eine Komponente zur Positionierung und/oder mechanischen Fixierung das reversible vertikale Kippen im Sinne einer Teildrehung oder das reversible vertikale Anheben im Sinne einer Verschiebung oder das reversible Gleiten mindestens der mindestens einen Komponente zur Übertragung eines Hochfrequenzsignals und der mindestens einen Komponente für den Flüssigkeitstransport in Bezug zu der mindestens einen mikroakustischen Komponente oder zu der mindestens einen Halterkomponente, um den Austausch der mikroakustischen Komponente realisieren zu können.

Weiterhin ist es vorteilhaft, dass die reversible Installation der mindestens einen mikroakustischen Komponente durch seitliches Gleiten, Einsetzen oder Verschieben der mikroakustischen Komponente in oder auf die Halterkomponente oder durch vertikales Auflegen oder Einsetzen der mikroakustischen Komponente in oder auf die Halterkomponente realisiert werden kann.

Vorteilhaft ist auch, wenn mindestens eine Steuer- und/oder Regelkomponente zur Steuerung und/oder Regelung des Austritts des Aerosolstrahls oder -jets aus der Aerosolaustrittskomponente oder aus der Komponente zur aerodynamischen Fokussierung des Aerosolstrahls oder -jets vorhanden ist, die an der Außenseite oder Innenseite der Aerosolaustrittskomponente oder der Komponente zur aerodynamischen Fokussierung des Aerosolstrahls oder -jets oder stromabwärts oberhalb der mikroakustischen Komponente angeordnet ist und/oder die Teil der Komponente für den Flüssigkeitstransport ist.

Es ist auch von Vorteil, wenn es sich bei der Steuer- und/oder Regelkomponente um einen drehbaren oder beweglichen Balken handelt, die im Aerosolstrahl oder -jet positioniert werden kann, und/oder um eine Komponente zum Verschließen der Öffnung oder der Öffnungen und/oder um eine Komponente, die eine Absaugvorrichtung enthält.

Im vorteilhaften Fall, dass die Steuer- und/oder Regelkomponente eine Absaugvorrichtung enthält, kann dadurch das Aerosol aufgefangen und aus der akustofluidischen Vorrichtung entfernt werden.

Ein großer Vorteil der erfindungsgemäßen Lösung besteht darin, dass die akustofluidische Vorrichtung zur Erzeugung von Aerosolen selbst sehr kleine Abmessungen von nur wenigen Zentimetern in allen räumlichen Dimensionen aufweist. Die geringe Komplexität der einzelnen Komponenten ermöglicht ein einfaches Plug'n'Play Setup mit anwendungsangepassten Komponenten, die leicht in bestehende Systeme integriert werden können und kürzere Installationszeiten, geringere Wartungsanforderungen und reduzierte Kosten ermöglichen. Hohe Zerstäubungseffizienz, kleine und einstellbare Tröpfchengrößen, ein kompaktes, einfaches Design und gute Reproduzierbarkeit der Aerosoleigenschaften machen die erfindungsgemäße akustofluidische Vorrichtung zur Erzeugung von Aerosolen ideal für verschiedene Anwendungen.

Die mikroakustische Aerosolerzeugung, die erfindungsgemäß eingesetzt wird, ist den etablierten Zerstäubungsmethoden (Ultraschall oder pneumatisch) in vielerlei Hinsicht überlegen. Neben weiteren, unten genannten Vorteilen, ermöglicht sie insgesamt ein völlig neues, kompaktes Design des Geräts mit geringerer Komplexität, bei dem insbesondere die Aerosolerzeugung und die Aerosolfokussierung in einer Einheit integriert sind. Komponenten wie eine Aerosolkammer, Aerosolzufuhrleitungen und virtueller Impaktor sind nicht mehr erforderlich. Dadurch werden der Platzbedarf des Geräts und die Anschaffungskosten erheblich reduziert - eine der wichtigsten Voraussetzungen für den Marktzugang und die Praktikabilität.

Der einfachere Aufbau der erfindungsgemäßen akustofluidischen Vorrichtung ohne Aerosolkammer und -schläuche führt auch zu einem geringeren Arbeits- und Ressourcenaufwand für die Einrichtung und Reinigung der Vorrichtung, was die Betriebskosten senkt. Die erfindungsgemäße akustofluidischen Vorrichtung ermöglicht somit auch eine höhere Effizienz bei der Aerosolerzeugung und eine bessere Ausnutzung der zu zerstäubenden Flüssigkeit.

Kleine Volumina (< 500 µl) der oft sehr teuren Druckflüssigkeiten reichen für die Zerstäubung aus und ein schneller Wechsel zwischen verschiedenen Flüssigkeiten ist unproblematisch und die Technologie bietet eine genauere Kontrolle über das Volumen der abgeschiedenen Materialien, was für Oberflächenbeschichtungsprozesse von Vorteil ist. Darüber hinaus können Flüssigkeiten in einem sehr breiten Viskositätsbereich verarbeitet werden und die Temperaturkontrolle der erfindungsgemäßen akustofluidischen Vorrichtung ermöglicht eine Temperaturkontrolle der Flüssigkeit. Für ein automatisiertes Aerosolabscheidungssystem kann die erfindungsgemäße akustofluidische Vorrichtung leicht in handelsübliche CAD-kompatible Fertigungs- oder Druckmaschinen, physikalische und/oder chemische Synthesegeräte, 3D-Druckplattformen oder Mehrachsroboter integriert werden.

Darüber hinaus ist es von besonderer Bedeutung, dass mit der erfindungsgemäßen Lösung der Winkel des Aerosolstrahls oder -jets in Bezug auf die mit dem Aerosol zu beschichtende Oberfläche durch Veränderung der Phasendifferenz und/oder der Phasenverschiebung einer Wellenform oder durch die der mikroakustischen Komponente zugeführte elektrische Leistung verändert werden kann. Die Änderung der Richtung des Aerosolstrahls oder -jets durch die erfindungsgemäße akustofluidischen Vorrichtung hat noch weitere verbesserte Effekte. Zum Beispiel kann die Millisekunden-Änderung der Richtung des Aerosolstrahls oder -jets als elektronisches Ventil verwendet werden, um den Zerstäubungsprozess ein- oder auszuschalten, was bei einer Druckanwendung entscheidend ist. Darüber hinaus kann die Steuerung der Zerstäubungsrichtung auch dazu verwendet werden, den Kondensationsbereich des Aerosols auf der Oberfläche zu kontrollieren oder zu maximieren.

Auch die Größe des Aerosolstrahls oder -jets und die Anfangsgeschwindigkeit des Aerosols können in-situ beeinflusst werden, um die Fokussierung des Aerosols und die Druckauflösung zu verbessern.

Indem ein Gas über die mindestens eine Transportgaskomponente in den Bereich der Aerosolerzeugung geleitet wird, kann dort eine gasförmige Umhüllung des Aerosols, d.h. ein Mantelgasstrom, realisiert werden. Durch die Beschleunigung des Aerosols, z.B. durch eine nachträgliche Verkleinerung des Auslassdurchmessers und/oder durch eine Erhöhung des Mantelgasdurchsatzes, kann ein Aerosolstrahl oder -jet erzeugt werden, der aus den Öffnungen der Aerosolauslasskomponente austritt und einen gezielten Auftrag des Aerosols auf eine Oberfläche realisieren kann.

Die erfindungsgemäßen Komponenten können mit bekannten Verfahren zur Herstellung komplexer Mikro- oder Makrostrukturen hergestellt werden, vorteilhafterweise mindestens teilweise mit additiven Fertigungsverfahren, Lithographie oder CNC-Techniken.

Ein weiterer besonderer Vorteil der erfindungsgemäßen Lösung ist, dass die verwendete Flüssigkeit und das daraus erzeugte Aerosol nicht in einem Kreislauf in die erfindungsgemäße akustofluidische Vorrichtung zurückgeführt werden müssen, da die Flüssigkeit im Wesentlichen direkt bei der akustofluidischen Wechselwirkung mit der akustischen Welle zu einem Aerosol zerstäubt und aus der akustofluidischen Vorrichtung herausgeführt wird.

Dies verhindert Verunreinigungen und erhöht die Stabilität sowohl der Flüssigkeit und/oder der Flüssigkeitszusammensetzung als auch die Qualität der Aerosolabscheidung. Die Auswirkungen von externen Temperaturschwankungen können ebenfalls vermieden oder begrenzt werden, da die mikroakustische Komponente, d.h. die Komponente, die mit der zu zerstäubenden Flüssigkeit in Kontakt ist, je nach Bedarf erwärmt oder gekühlt werden kann. Die Kombination mit einer Wärmetransportkomponente, die auch ein Peltier-Element enthalten kann, und mit mindestens einem Temperatursensor ermöglicht eine direkte Überwachung und Kontrolle der Temperaturschwankungen, wodurch die Verträglichkeit der erfindungsgemäßen akustofluidischen Vorrichtung mit empfindlichen Flüssigkeiten, die Effizienz und die Flüssigkeitsqualität während des Gebrauchs überwacht und auch verbessert werden können.

In der erfindungsgemäßen Lösung werden akustische Oberflächenwellen vorteilhaft eingesetzt, um Flüssigkeiten in ein Aerosol aus präzisen Tröpfchen mit einer Größe von weniger als 30 µm zu zerstäuben, was die für die Abscheidung relevante Qualität des Aerosols erheblich verbessert.

Durch den Einsatz der erfindungsgemäßen akustofluidischen Vorrichtung zur Erzeugung von Aerosolen in der Drucktechnik werden Vorteile gegenüber herkömmlichen Aerosolquellen, Tintenstrahl- oder anderen Druckkopftechnologien erzielt, wie z.B. eine deutlich verbesserte Skalierbarkeit, Kosteneffizienz, Materialverträglichkeit und Energieeffizienz, Aerosol- oder Ablagerungsrate, Aerosolauflösung und/oder Vielseitigkeit.

Die erfindungsgemäße akustofluidische Vorrichtung realisiert eine Zerstäubung der Flüssigkeit in ein kompaktes Aerosol mit kleinem Öffnungswinkel, wodurch eine deutlich verbesserte Fokussierung des Aerosolstrahls erreicht werden kann.

Ein weiterer Vorteil der erfindungsgemäßen akustofluidischen Vorrichtung ist, dass alle Komponenten in einem gasdichten Gehäuse, z.B. einem Standard-Rohr- und Flanschsystem, angeordnet werden können, was Druckabfälle und die Freisetzung von Nanopartikeln verhindern kann und eine einfache Integration in Synthesegeräte ermöglicht.

Weitere Vorteile der erfindungsgemäßen Lösung sind
- einfacher Austausch der mikroakustischen Komponente bei gleichzeitiger Sicherstellung einer zuverlässigen Flüssigkeitsversorgung und einer elektrischen Hochfrequenzverbindung, und
- die Verhinderung der Rezirkulation der Flüssigkeit und der Beeinträchtigung der Flüssigkeitseigenschaften durch eine längere Einwirkung des Schallfeldes.

Die Erfindung wird im Folgenden anhand eines Ausführungsbeispiels näher erläutert.

Dabei zeigt
- Fig. 1: die schematische Struktur von einer erfindungsgemäßen akustofluidischen Vorrichtung zur Erzeugung von Aerosolen zur Verwendung als Druckkopf
und
- Fig. 2: den schematischen Aufbau einer erfindungsgemäßen akustofluidischen Vorrichtung zur Erzeugung von Aerosolen integriert in eine röhrenförmige chemische Synthesevorrichtung

### Beispiel 1

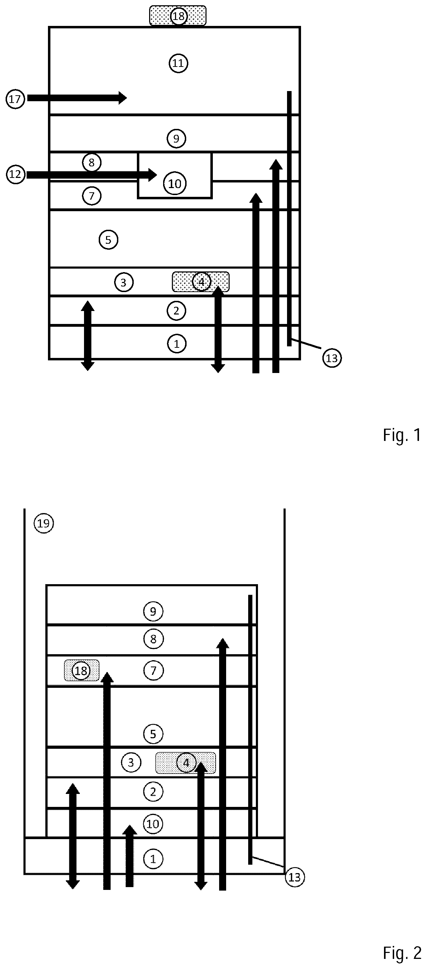

Eine akustofluidische Vorrichtung zur Erzeugung von Aerosolen gemäß Fig. 1 weist eine Grundplatte 1 auf, auf der alle Komponenten positioniert sind. Dabei sind die Zu- und/oder Ableitungen für Gase 12, 17 seitlich mit der akustofluidischen Vorrichtung verbunden und die elektrische Hochfrequenzsignalleitung 6, die elektrische Signalleitung 16, die Zu- und/oder Ableitungen für Flüssigkeiten 15 und für Wärme 14 von hinten über die Grundplatte 1 mit der akustofluidischen Vorrichtung verbunden.

Auf der Vorderseite der Grundplatte 1 ist eine Komponente zum Wärmetransport 2 angeordnet, die einen mit Kühlflüssigkeit gefüllten Hohlraum umfasst und mit zwei Zu- und Ableitungen für Wärme 14 verbunden ist, wodurch ein geschlossenes System mit zwei flüssigkeitsgefüllten Schläuchen realisiert ist, welches mit einem peripheren Kühler verbunden ist und im geschlossenen Kreislauf warme Kühlflüssigkeit von der Wärmetransportkomponente 2 zum Kühler und kalte Kühlflüssigkeit zur Wärmetransportkomponente 2 transportieren.

Durch diese Anordnung wird die akustofluidische Vorrichtung mittels einer Flüssigkeitskühlung aus peripheren Komponenten aktiv gekühlt.

Die mikroakustische Komponente 5 ist auf der Halterkomponente 3 oberhalb der Komponente zur Wärmeübertragung 2 angeordnet.

Die mikroakustische Komponente 5 ist eine Kartusche, die einen mikroakustischen Chip enthält, der mit einem wärmeleitenden, doppelseitig klebenden Silikonband mit einer Dicke von 200 µm als Halterkomponente 3 auf einer 2 mm dicken Platte der mikroakustischen Komponente 5 aus Kupfer befestigt ist. Auf dem mikroakustischen Chip werden stehende akustische Rayleigh-Oberflächenwellen (sSAWs) über zwei gegenüberliegende Interdigitalwandler (IDT) (Typ λ/4, 90 µm Wellenlänge, 0,5 mm Apertur, 6 mm Abstand dazwischen, angepasst an eine Impedanz von 50 Ω durch Verwendung von jeweils 46 Fingerelektrodenpaaren) angeregt. Die Platte, auf der der mikroakustische Chip positioniert ist, besteht aus einseitig poliertem, transparentem Lithiumniobat (128°YX) und die Interdigitalwandler-Elektroden bestehen aus einem Schichtaufbau aus einer 10 nm dicken Schicht aus Ti, gefolgt von einer 290 nm dicken Schicht aus Al.

Auf dem mikroakustischen Chip befindet sich 500 nm Siliziumdioxid als Schutzschicht. Auf dem mikroakustischen Chip ist eine teilweise offene, tröpfchenförmige mikrofluidische flüssigkeitsführende Struktur mit innenliegendem Kanal (Kanalbreite = 100 µm, Wandbreite am Auslass = 50 µm) vorhanden, die aus zwei Schichten aus 50 µm dickem Epoxid-Trockenfilmresist (Wandschicht + Deckschicht) besteht, um die Flüssigkeitszufuhr auf der Oberfläche des mikroakustischen Chips zu realisieren.

Als Komponente für den Flüssigkeitstransport 7 ist eine Platte aus PEEK-Kunststoff auf der mikroakustischen Komponente 5 und der Halterkomponente 3 angeordnet und über einen O-Ring flüssigkeitsdicht mit der teilweise offenen, tröpfchenförmigen mikrofluidischen flüssigkeitsführenden Struktur der mikroakustischen Komponente 5 verbunden.

Ein Temperatursensor 4 ist in einem gebohrten Loch innerhalb der Halterkomponente 3 angeordnet und über eine elektrische Signalleitung 16 mit einem peripheren Temperaturlogger verbunden. Die Komponente für den Flüssigkeitstransport 7 und die Komponente zur Übertragung eines Hochfrequenzsignals 8 wurden oben auf der Halterkomponente 3 montiert und mit Schrauben befestigt, wodurch die mikroakustische Komponente 5 elektrisch und flüssigkeitsführend mit der elektrischen Signalleitung 16 und mit der Zuleitung für Flüssigkeit 15 verbunden ist.

Dabei haben die Komponente für den Flüssigkeitstransport 7 und die Komponente zur Übertragung eines Hochfrequenzsignals 8 in ihrer Mitte ein Loch, durch das das Aerosol die akustofluidische Vorrichtung verlassen kann. Eine Transportgaskomponente 10 ist teilweise innerhalb dieses Lochs angebracht, um das Transportgas Stickstoff über die Zuleitung für Gas 12 mit einer Flussrate von 167 sccm zu transportieren und in einem vertikalen Abstand von 10 mm zur flüssigkeitsbeaufschlagten Oberfläche der mikroakustischen Komponente 5 auf den mikroakustischen Chip als koaxialen Gasstrom um den erzeugten Aerosolstrahl oder -jet einzuleiten. Die Transportgaskomponente 10 behindert dabei aber nicht den Austritt des Aerosols.

Auf der Transportgaskomponente 10 ist eine Aerosolauslasskomponente 9 mit einem zentralen Loch von 5 mm Durchmesser als Aerosolauslass angebracht.

Darauf wird eine Komponente zur Fokussierung des Aerosols 11 angeordnet, um die Aerosolführung zu verbessern und die Geschwindigkeit des Aerosols mit Hilfe von Stickstoff über einer Zuleitung für Gas 17 zu erhöhen. Das Aerosol wird durch eine auf dem Luer-Lock-Gewinde montierte Kunststoffspitze aus der akustofluidischen Vorrichtung befördert. Die Kunststoffspitze ist mit einer Aerosolführung (Nordson EFD Optimum^{®} SmoothFlow^{™} mit einem Düsenspitzen-Innendurchmesser von 800 µm) ausgerüstet.

Eine Steuer- und/oder Regelkomponente 18, die aus einem um 45° drehbaren Balken besteht, ist beweglich an der Aerosolfokussierungskomponente 11 angebracht. Sie kann von einer seitlichen Position in eine Position gedreht werden, die den Auslass der Düsenspitze abdeckt und damit den Aerosolaustritt verhindert.

Alle Komponenten werden mit drei Komponenten zur Positionierung und mechanischen Fixierung 13 aneinander befestigt und mit 3 Schrauben zusammengehalten.

Die elektrischen Signale werden mit der Betriebsfrequenz der IDTs über zwei elektrische Hochfrequenzsignalleitungen 6 mit SMA- und MMCX-Verbindern von einer zweikanaligen Signalquelle geliefert. Die Flüssigkeit wird über einen PTFE-Schlauch über die Zuleitung für Flüssigkeit 15 von einer peripheren Spritzenpumpe in die Komponente für den Flüssigkeitstransport 7 und damit auf die mikroakustische Komponente 5 eingeleitet.

Die akustofluidische Vorrichtung trägt damit Silber-Tinte (JS-426 Novacentrix) (1:24 mit DI-Wasser) in Linien von mehreren Millimetern Länge und einer Breite von ≈ 130 µm auf ein Lithiumniobat-Substrat mit einer Abscheidungsrate von 5 mm/s auf. Die Zerstäubung erfolgt bei einer Flüssigkeitsvolumenstrom von 20 µl/min, einer Leistung von 4,4 W und einer Signalfrequenz von 42,8 MHz.

### Bezugszeichenliste:

- 1: Grundplatte
- 2: Wärmetransportkomponente
- 3: Halterkomponente
- 4: Temperatursensor
- 5: Mikroakustische Komponente
- 6: Elektrische Hochfrequenzsignalleitung
- 7: Komponente für den Flüssigkeitstransport
- 8: Komponente zur Übertragung eines Hochfrequenzsignals
- 9: Aerosolauslasskomponente
- 10: Transportgaskomponente
- 11: Aerosolfokussierungskomponente
- 12: Zuleitung für Gas
- 13: Komponente zur Positionierung und/oder mechanischen Fixierung
- 14: Zu- und/oder Ableitungen für Wärme
- 15: Zuleitung für Flüssigkeiten
- 16: Elektrische Leitung und Signalleitung
- 17: Zuleitung für Gas
- 18: Steuer- und/oder Regelkomponente
- 19: Röhrenförmiges Gehäuse

## Patentansprüche

1. Akustofluidische Vorrichtung zur Erzeugung von Aerosolen, die mindestens enthält
- mindestens eine Aerosolauslasskomponente mit mindestens einer Öffnung, aus der ein gerichteter Aerosolstrahl oder -jet austritt, und
- mindestens eine Halterkomponente für mindestens eine mikroakustische Komponente, und
- mindestens je eine Zu- und/oder Ableitung für Gase, Flüssigkeiten und Wärme, mindestens eine elektrische Leitung und/oder elektrische Signalleitung und mindestens eine elektrische Hochfrequenzsignalleitung, und
- mindestens eine Wärmetransportkomponente, die den Wärmetransport von und/oder zu der mindestens einen Halterkomponente realisiert, und die mit der mindestens einen Zu- und/oder Ableitung für Wärme verbunden ist, und
- mindestens einen Temperatursensor, der thermisch leitend mit der Halterkomponente und/oder der Wärmetransportkomponente und elektrisch leitend mit der mindestens einen elektrischen Signalleitung verbunden ist, und
- mindestens eine mikroakustische Komponente mit einer lateralen Abmessung von maximal 4 × 4 cm² mit mindestens einem Schallwandler und/oder elektroakustischen und/oder piezoelektrischen Wandler für die Anregung akustischer Wellen im Frequenzbereich von 1 bis 500 MHz, und
- mindestens eine Komponente zur Übertragung eines Hochfrequenzsignals, die elektrisch leitend und hochfrequenztauglich mit der mindestens einen elektrischen Hochfrequenzsignalleitung und mit der mindestens einen mikroakustischen Komponente verbunden ist, und die die Übertragung eines Hochfrequenzsignals über die mindestens eine elektrische Hochfrequenzsignalleitung zu und/oder von der mindestens einen mikroakustischen Komponente realisiert, und
- mindestens eine Komponente für den Flüssigkeitstransport, die mit der mindestens einen Zu- und/oder Ableitung für Flüssigkeiten und mit der mindestens einen mikroakustischen Komponente flüssigkeitsführend verbunden ist, und mit der mindestens die Zuführung von Flüssigkeiten mit einem Volumenstrom zwischen 0,1 µl/min und 5 ml/min auf die Oberfläche der mindestens einen mikroakustischen Komponente realisiert ist, und
- mindestens eine Transportgaskomponente, die mit der mindestens einen Zu- und/oder Ableitung für Gase verbunden ist, wobei mit der Transportgaskomponente mindestens die Zufuhr mindestens eines Gases in die unmittelbarer Nähe des Bereichs der akustofluidischen Wechselwirkung zwischen der akustischen Welle und der Flüssigkeit zur Aerosolerzeugung auf der flüssigkeitsbeaufschlagten Oberfläche der mindestens einen mikroakustischen Komponente realisiert ist, und
- mindestens eine Komponente zur Positionierung und/oder mechanischen Fixierung der mindestens einen Komponente zur Übertragung eines Hochfrequenzsignals und der mindestens einen Komponente für den Flüssigkeitstransport in Bezug zu der mindestens einen mikroakustischen Komponente oder zu der mindestens einen Halterkomponente, wobei die mindestens eine Komponente zur Positionierung und/oder mechanischen Fixierung eine unabhängige Komponente und/oder eine Komponente der Halterkomponente und/oder der Komponente für den Flüssigkeitstransport und/oder der Aerosolauslasskomponente und/oder einer anderen Komponente ist.

2. Akustofluidische Vorrichtung nach Anspruch 1, bei der die mindestens eine mikroakustische Komponente die Anregung mindestens einer akustischen Welle, vorteilhaft einer akustischen Rayleigh-Oberflächenwelle und/oder einer akustischen Sezawa-Oberflächenwelle, und/oder einer akustischen Plattenwelle, vorteilhaft einer symmetrischen und/oder asymmetrischen Lamb-Welle, und/oder einer akustischen Volumenwelle, vorteilhaft einer Longitudinal- und/oder Scherwelle, und/oder einer stehenden akustischen Oberflächenwelle und/oder einer Körper- und/oder Flüssigkeitsschallwelle realisiert.

3. Akustofluidische Vorrichtung nach Anspruch 1, bei der die mindestens eine Komponente für den Flüssigkeitstransport mit mindestens einer Flüssigkeitspumpe und/oder mindestens einem Flüssigkeitsreservoir verbunden ist oder mindestens eine Flüssigkeitspumpe und/oder mindestens ein Flüssigkeitsreservoir und/oder mindestens eine Mikropumpe und/oder mindestens eine elektroosmotischen Pumpe und/oder mindestens ein Flüssigkeitsventil und/oder mindestens eine sensorische und/oder analytische Komponente beinhaltet.

4. Akustofluidische Vorrichtung nach Anspruch 1, bei der die mindestens eine Komponente zur Positionierung und/oder mechanischen Fixierung ein reversibles vertikales Kippen im Sinne einer Teildrehung oder ein reversibles vertikales Anheben im Sinne einer Verschiebung oder ein reversibles Gleiten der mindestens einen Komponente zur Übertragung eines Hochfrequenzsignals und der mindestens einen Komponente für den Flüssigkeitstransport in Bezug zu der mindestens einen mikroakustischen Komponente oder zu der mindestens einen Halterkomponente zum Austausch der mikroakustischen Komponente realisiert.

5. Akustofluidische Vorrichtung nach Anspruch 4, bei der die reversible Installation der mindestens einen mikroakustischen Komponente durch seitliches Gleiten, Einsetzen oder Verschieben der mindestens einen mikroakustischen Komponente in die oder auf der Halterkomponente oder durch vertikales Auflegen oder Einsetzen der mindestens einen mikroakustischen Komponente auf oder in die Halterkomponente realisiert ist.

6. Akustofluidische Vorrichtung nach Anspruch 1, bei der die mindestens eine Wärmetransportkomponente mindestens ein Wärmerohr und/oder einen Kühlkörper mit oder ohne Komponenten zur Verbesserung der Gaskonvektion und/oder mit damit verbundener Flüssigkeitskühlung und/oder mindestens ein Peltier-Element mit einem passiven Rippenkühler mit oder ohne Komponenten zur Verbesserung der Gaskonvektion und/oder mindestens ein Peltier-Element mit einem Kühlkörper mit damit verbundener Flüssigkeitskühlung enthält oder damit verbunden ist.

7. Akustofluidische Vorrichtung nach Anspruch 1, bei der die mindestens eine mikroakustische Komponente ein mikroakustischer Chip oder eine mikroakustische Kartusche ist, wobei die mikroakustische Kartusche aus mindestens einem mikroakustischen Chip auf mindestens einer Platte oder Folie aus einem Material mit hoher Wärmeleitfähigkeit besteht, und wobei zusätzliche Schichten aus hochwärmeleitfähigem Material mindestens zwischen Chip und Platte oder Folie und/oder auf der dem Chip abgewandten Seite der Platte oder Folie vorhanden sind, wobei vorteilhafterweise eine hochwärmeleitende Verbindung zwischen dem mindestens einen mikroakustischen Chip und der Platte oder Folie mit einem Klebstoff und/oder einer haftungsfördernden Flüssigkeit und/oder ein- oder doppelseitigem Klebeband und/oder metall- und/oder keramikgefüllten Polymeren, Epoxiden und/oder Pasten und/oder einem Klebeband oder Klebestreifen und/oder mittels eines nichtpermanenten Klebstoffs realisiert ist.

8. Akustofluidische Vorrichtung nach Anspruch 1, bei der die mindestens eine mikroakustische Komponente und/oder die mindestens eine Komponente für den Flüssigkeitstransport mindestens ein mikrofluidisches und/oder flüssigkeitsleitendes Strukturelement aufweist und die mindestens eine Komponente für den Flüssigkeitstransport mit der mindestens einen mikroakustischen Komponente über mindestens ein mikrofluidisches und/oder flüssigkeitsleitendes Strukturelement flüssigkeitsleitend verbunden ist, wobei vorteilhafterweise mindestens ein Mikrokanal und/oder ein Verbindungselement und/oder ein Dichtungselement und/oder ein Reservoir und/oder eine Mikromembran und/oder ein nadelförmiges Strukturelement und/oder ein schwamm- oder gewebeförmiges Element als mikrofluidisches und/oder flüssigkeitsleitendes Strukturelement vorhanden ist.

9. Akustofluidische Vorrichtung nach Anspruch 1, bei der mit der mindestens einen Transportgaskomponente ein koaxiales Einströmen eines Gases in den Bereich der akustofluidischen Wechselwirkung zwischen der akustischen Welle und der Flüssigkeit zur Umhüllung des erzeugten Aerosolstrahls oder -jets erzeugt ist, wobei die Stelle des Austritts des Gases aus der Transportgaskomponente höchstens 30 mm, vorteilhafterweise < 15 mm, noch vorteilhafter < 5 mm, entfernt angeordnet ist von der flüssigkeitstragenden Oberfläche der mindestens einen mikroakustischen Komponente in Richtung des Volumenstromvektors des Aerosolstrahls oder -jets.

10. Akustofluidische Vorrichtung nach Anspruch 9, bei der der rotationssymmetrische Volumenstromvektor des koaxial einströmenden Gases um nicht mehr als 45°, vorteilhafterweise um nicht mehr als 30°, vom Volumenstromvektor des Aerosolstrahls oder -jets, oder von der Oberflächennormalen der flüssigkeitstragenden Oberfläche der mindestens einen mikroakustischen Komponente abweicht.

11. Akustofluidische Vorrichtung nach Anspruch 1, bei der stromabwärts von der mindestens einen Transportgaskomponente oder nach der mindestens einen Öffnung in der Aerosolauslasskomponente in Richtung des Volumenstromvektors des Aerosolstrahls oder -jets mindestens eine Komponente zur aerodynamischen Fokussierung des Aerosolstrahls oder -jets mit einem Gasmantel und/oder zur Erhöhung der Geschwindigkeit des Aerosolstrahls oder -jets vorhanden ist, die mindestens eine weitere Zu- und/oder Ableitung für Gase aufweist.

12. Akustofluidische Vorrichtung nach Anspruch 1, bei der ein zusätzliches rohrförmiges Gehäuse die akustofluidische Vorrichtung umschließt, welches aus Metall, Polymer, Keramik oder Glas besteht, und welches an mindestens einem Rohrende mindestens einen Flansch der Bauart ISO-K, ISO-KF, ISO-F, CF oder COF für die Zu- und Ableitungen und elektrische Signalleitungen und elektrische Hochfrequenzsignalleitungen aufweist.

13. Akustofluidische Vorrichtung nach Anspruch 1, bei der die Öffnung oder die Öffnungen in der Aerosolauslasskomponente Abmessungen von nicht größer als 5 mm aufweisen und/oder eine kreisförmige, koaxiale und/oder ovale Form haben und/oder Mehrfachöffnungsauslässe sind und/oder Luer- und/oder metrische und/oder zöllige Gewinde aufweisen.

14. Akustofluidische Vorrichtung nach Anspruch 1, bei der die mindestens eine Steuer- und/oder Regelkomponente zur Steuerung und/oder Regelung des Austritts des Aerosolstrahls oder -jets aus der mindestens einen Aerosolauslasskomponente oder aus der mindestens einen Komponente zur aerodynamischen Fokussierung des Aerosolstrahls oder -jets vorhanden ist, die an der Außenseite oder Innenseite der mindestens einen Aerosolauslasskomponente oder der mindestens einen Komponente zur aerodynamischen Fokussierung des Aerosolstrahls oder -jets oder stromabwärts oberhalb der mindestens einen mikroakustischen Komponente angeordnet ist und/oder Teil der mindestens einen Komponente für den Flüssigkeitstransport ist.

15. Akustofluidische Vorrichtung nach Anspruch 14, bei der die mindestens eine Steuer- und/oder Regelkomponente ein drehbarer oder beweglicher Balken ist, der im Aerosolstrahl oder -jet positioniert ist und/oder eine Komponente zum Verschließen der Öffnung oder der Öffnungen ist und/oder eine Komponente ist, die eine Absaugvorrichtung enthält.
